# EUROPÄISCHE PATENTSCHRIFT

(11) **EP 1 575 455 B1**
(45) Veröffentlichungstag und Bekanntmachung des Hinweises auf die Patenterteilung: **20.02.2008**
(21) Anmeldenummer: 03782141.0
(22) Anmeldetag: 09.12.2003
(51) Int. Cl.: A61F 2/38, A61F 2/30

(54) **KÜNSTLICHES GELENK**
ARTIFICIAL JOINT
ARTICULATION PROTHETIQUE

(30) Priorität: 10.12.2002 DE 10257774
(43) Veröffentlichungstag der Anmeldung: 21.09.2005
(73) Patentinhaber: HJS Gelenk System GmbH, 81925 München (DE)
(72) Erfinder: ABICHT, Christian, 07745 Jena (DE); ADAM, Peter, 85221 Dachau (DE); KUBEIN-MEESENBURG, Dietmar, 37075 Göttingen (DE); NÄGERL, Hans, 37130 Gleichen (DE)
(74) Vertreter: Scheffler, Jörg
(86) Internationale Anmeldenummer: PCT/DE2003/004025
(87) Internationale Veröffentlichungsnummer: WO 2004/052244

(56) Entgegenhaltungen:
- WO-A-95/22944
- WO-A-02/078565
- US-A- 4 878 916

## Beschreibung

Die Erfindung betrifft ein künstliches Gelenk mit einem Gelenkplateau und einer Gelenkauflage, die mittels einer durch eine Ausnehmung und einen darin eingesetzten Vorsprung bestimmte Kontaktfläche miteinander verbunden sind, wobei der Vorsprung gegenüber der Ausnehmung bei Körpertemperatur des Patienten ein Übermaß aufweist und mittels einer Temperaturdifferenz des Vorsprunges oder der Ausnehmung gegenüber der Körpertemperatur des Patienten in die Ausnehmung einsetzbar ist. Weiterhin betrifft die Erfindung ein Verfahren zur Herstellung einer Verbindung in einem solchen künstlichen Gelenk.

Ein solches künstliches Gelenk ist beispielsweise bereits durch die US 487 8916 bekannt, die den nächsten Stand der Technik darstellt. Das künstliche Gelenk hat ein dabei ein rotationssymmetrisches Gelenkplateau und eine kugelschalenförmige Gelenkauflage, die mittels einer Schrumpfverbindung miteinander verbunden sind. Die Gelenkauflage weist in dem Gelenkplateau bei Körpertemperatur des Patienten ein Übermaß auf und ist daher ausschließlich mittels einer Temperaturdifferenz gegenüber der Körpertemperatur des Patienten in die Ausnehmung einsetzbar.

Gattungsgemäße künstliche Gelenke sind auch durch die US 58 58 020 sowie die FR 24 93 139 A1 bekannt.

Als nachteilig erweist sich bei den bekannten Gelenken der genannten Art, dass die Belastbarkeit des künstlichen Gelenkes nicht dauerhaft sichergestellt werden kann. Insbesondere sind Beschädigungen der Gelenkteile nicht zuverlässig auszuschließen.

Durch die EP 04 95 340 A1 ist auch weiterhin bereits ein modularer Bausatz für den Tibiateil einer Kniegelenkprothese bekannt, der aus einem Tibiaplateau mit einem Zapfen und aus einer Gelenkauflage aus Polyethylen besteht. In das Tibiaplateau sind unterschiedlich große Stämme einsetzbar und unterschiedlich hohe Gelenkauflagen sind in einer Ebene des Tibiaplateaus allseitig geführt. Zur Fixierung der Gelenkauflage wird diese in die Ebene des Tibiaplateaus durch eine Schwenkbewegung eingebracht. Als nachteilig hat es sich dabei jedoch erwiesen, dass die dadurch erzielbare Belastbarkeit vergleichsweise gering ist und zudem die so gebildete Schnappverbindung im Gebrauchszustand einen Hohlraum zwischen der Gelenkauflage und dem Tibiaplateau ausspart.

Es ist ferner auch durch die CH-PS 66 73 83 ein Tibiateil einer Kniegelenkprothese beschrieben, die aus einem Tibiaplateau mit Zapfen und aus einer Gelenkauflage besteht. Der Einbau dieser Kniegelenkprothese erfordert jedoch eine individuelle Vorbearbeitung, die wesentlich vom Geschick des Operateurs abhängig ist.

Der Erfindung liegt die Aufgabe zugrunde, eine Möglichkeit zu schaffen, eine Kniegelenkprothese der eingangs genannten Art derart auszuführen, dass dadurch die Belastbarkeit insbesondere Dauerbelastbarkeit über einen langen Zeitraum hinweg wesentlich verbessert ist. Weiterhin soll ein Verfahren zur Herstellung einer solchen Kniegelenkprothese geschaffen werden.

Die erstgenannte Aufgabe wird erfindungsgemäß mit einer Kniegelenkprothese gemäß den Merkmalen des Anspruchs 1 gelöst. Die weitere Ausgestaltung der erfindungsgemäßen Kniegelenkprothese ist den Unteransprüchen 2 bis 15 zu entnehmen.

Erfindungsgemäß ist also ein künstliches Gelenk vorgesehen, die Kontaktfläche bzw. das Übermaß ortsabhängig unterschiedlich derart bemessen ist, dass aufgrund der auf den Vorsprung wirkenden Fixierkräfte bei Körpertemperatur des Patienten in der Gelenkauflage und/oder Gelenkplateaus ein Spannungszustand realisiert ist, durch den die Belastbarkeit und/oder Haltbarkeit des Gelenkes erhöht ist. Die Erfindung geht dabei von der überraschenden Erkenntnis aus, dass die bei dem künstlichen Gelenk zur Fixierung bestimmte und mittels der Temperaturdifferenz realisierte thermische Schrumpfverbindung zugleich auch in vorteilhafter Weise dazu genutzt werden kann, mittels der auf diese Weise eingebrachten Fixierkräfte und dadurch erzeugten Spannungen die Belastbarkeit des Gelenkes zu erhöhen. Hierzu werden die Spannungen in optimaler Weise derart eingestellt, dass diese der lastbedingten Krafteinwirkung entgegenwirken. Beispielsweise kann auf diese Weise ein mehrdimensionaler Spannungszustand erzeugt werden, bei dem die Gelenkauflage lediglich durch Druckkräfte belastet wird, hingegen die Einwirkung von Zugkräften aufgrund der Vorspannung ausgeschlossen ist.

Hierzu wird nach einer besonders vorteilhaften Ausführungsform der Erfindung aufgrund der Fixierkräfte ein gezielter Druckzustand in der Gelenkauflage erzeugt, welcher einer äußeren Krafteinwirkung unter Belastung entgegenwirkt.

Dabei ist es besonders praxisgerecht, wenn die Kontaktfläche derart ausgeführt ist, dass der Spannungszustand oder der Druckzustand hinsichtlich des Betrages und/oder und der Richtung einstellbar ist, um auf diese Weise eine optimale Anpassung des Gelenkes an den Patienten und dessen individuelle Anforderungen erfüllen zu können.

Dabei erweist sich eine Abwandlung als besonders belastbar, bei welcher der Betrag des aufgrund der Fixierkräfte eingebrachten Druckzustandes größer als der Betrag einer äußeren Krafteinwirkung ist. Hierdurch treten auch bei großen äußeren Krafteinwirkungen in der Gelenkauflage keine Zugkräfte auf. Die Belastbarkeit hinsichtlich äußerer Druckkräfte ist demgegenüber jedoch erhöht, so dass hierdurch eine Erhöhung der Belastbarkeit einhergeht.

Eine besonders praxisrelevante Gestaltung wird auch dann erreicht, wenn der Druck- bzw. Spannungszustand in Abhängigkeit der Hauptbelastungsebene des künstlichen Gelenkes einstellbar ist. Hierdurch ist es möglich, einen an die Belastung des Gelenkes durch den Patienten angepassten Spannungszustand bereits bei der Fixierung der Gelenkauflage an dem Gelenkplateau zu erreichen. Hierdurch kann aufgrund der auf diese Weise optimierten Anpassung des Gelenkes eine weitere Steigerung der Belastungsgrenzen insbesondere bei an sich bekannten Werkstoffen erreicht werden, ohne zu diesem Zweck die äußeren Abmessungen der Bauelemente ändern zu müssen.

In besonders vorteilhafter Weise wird erreicht, dass der Spannungszustand durch eine einer äußeren Krafteinwirkung entgegenwirkende Vorspannung gegeben ist.

Der Spannungszustand könnte bereits herstellerseitig weitgehend vorbestimmt sein. Um jedoch eine Anpassung an den Patienten in besonders vorteilhafter Weise realisieren zu können ist im Bereich der Kontaktfläche ein zur Einstellung des Spannungszustand vorgesehenes Zwischenelemente vorgesehen, durch das der Spannungszustand ortsabhängig variabel eingestellt werden kann.

Beispielsweise ist hierzu das Element in unterschiedlichen Positionen festlegbar, um so eine einfache Einstellbarkeit durch den Operateur zu ermöglichen.

Als besonders praxisgerecht erweist sich dabei auch eine Abwandlung bei der das Element als eine Schale ausgeführt ist und eine Festlegung des Gelenkplateaus gegenüber der Gelenkauflage in unterschiedlichen Winkelstellungen gestattet.

Zu diesem Zweck erweist sich eine Ausgestaltung als besonders vorteilhaft, bei der die Ausnehmung eine den Vorsprung formschlüssig fixierende Hinterschneidung aufweist, die durch eine Kontur oder eine Topographie der Gelenkäuflage, insbesondere der Materialstärke, bestimmt ist, weil dadurch die auftretenden Fixierkräfte in verschiedenen Bereichen unterschiedlich eingestellt werden können. Weiterhin könnten unterschiedliche Übermaße in Abhängigkeit der Funktionsebenen des Gelenkes vorgesehen werden. Es kann also der Spannungszustand in dem Gelenk insbesondere hinsichtlich der erwartungsgemäßen Belastung wesentlich optimiert werden und zugleich die Lebensdauer erhöht werden. Der in die Ausnehmung eingesetzte Vorsprung ist dabei mittels der Schrumpfverbindung kraft- und formschlüssig fixiert. Daher ist auch die Fixierung einer nicht rotationssymmetrischen, beispielsweise nierenförmigen Gelenkauflage uneingeschränkt geeignet. Beispielsweise wird durch eine gegenüber der Fügerichtung geneigte Kontaktfläche eine Vorspannkraft erzeugt, durch die zugleich auch eine Spaltbildung zwischen dem Gelenkplateau und der Gelenkauflage zuverlässig ausgeschlossen werden.
Eine besonders vorteilhafte Ausführungsform der Erfindung, wird auch dann erreicht, wenn das Gelenkplateau und die Gelenkauflage jeweils einen unterschiedlichen Wärmeausdehnungskoeffizienten aufweisen. Hierdurch führt eine gemeinsame Erwärmung oder Abkühlung des Gelenkplateaus zusammen mit der Gelenkauflage zu einer unterschiedlichen Wärmeausdehnung bzw. Kälteschrumpfung, so dass sich ein Differenzmaß einstellt. Es ist daher nicht erforderlich, lediglich die Ausnehmung zu erwärmen und / oder den Vorsprung abzukühlen und in dem so eingestellten Zustand zusammenzufügen.

Dabei erweist es sich als besonders praxisnah, wenn das Gelenkplateau und die Gelenkauflage konturbündig miteinander verbunden sind. Hierdurch können unerwünschte Vorsprünge vermieden und die Belastbarkeit auch über einen langen Zeitraum hinweg wesentlich verbessert werden. Die äußeren Abmessungen, beispielsweise die Querschnittsfläche des Gelenkplateaus und der Gelenkauflage stimmen dabei insbesondere identisch überein.

Besonders einfach ist hingegen eine Abwandlung der erfindungsgemäßen Kniegelenkprothese, bei der eine Kontaktfläche zwischen dem Vorsprung und der Ausnehmung mit einer die Kraftübertragung verbessernden Oberflächenbeschaffenheit, insbesondere Rauhigkeit oder Strukturierung, ausgestattet ist. Hierdurch kann die Belastbarkeit der Verbindung durch eine verbesserte Haftreibung deutlich erhöht werden. Die Kontaktfläche ist hierzu beispielsweise mit einer Strukturierung ausgestattet.

Als besonders praxisgerecht erweist sich dabei auch eine Ausgestaltung der Erfindung, bei welcher der Gelenkauflage ein Vorsprung aus Polyethylen und dem Gelenkplateau eine Ausnehmung aus einem Metall zugeordnet ist. Hierdurch wird ein optimales Verhältnis der Wärmedehnungskoeffizienten erreicht, so dass bereits eine Temperaturdifferenz von ca. 10°C zu einer Dehnungsdifferenz von ca. 0,1 mm führt. Die Handhabung des Gelenkplateaus und der Gelenkauflage zur Montage wird dadurch erleichtert und gestattet daher eine entsprechend der jeweiligen individuellen Anforderungen des Patienten vorzunehmende Auswahl der geeigneten Gelenkauflage während der Behandlung.

Weiterhin hat es sich als besonders zweckmäßig erwiesen, wenn der Vorsprung und die Ausnehmung an einer umlaufende Kontaktfläche gegeneinander liegen, die einen stetigen Verlauf hat. Hierdurch wird eine gleichmäßige Krafteinleitung über die gesamte Länge der Kontaktfläche realisiert, wodurch die Belastbarkeit der Verbindung weiter verbessert werden kann. Dabei weist nach einer weiteren besonders günstigen Abwandlung der vorliegenden Erfindung das Gelenkplateau die Ausnehmung und die Gelenkauflage den Vorsprung auf.

Die zweitgenannte Aufgabe, ein Verfahren zur Herstellung eines insbesondere als Kniegelenkprothese ausgeführten künstlichen Gelenks zu schaffen, bei dem ein Gelenkplateau mit einer Gelenkauflage mittels einer durch eine Ausnehmung und einen darin eingesetzten Vorsprung gebildeten Kontaktfläche verbunden wird, wird erfindungsgemäß dadurch gelöst, dass die Kontaktfläche bzw. das Übermaß ortsabhängig unterschiedlich derart bemessen werden, dass aufgrund der auf den Vorsprung wirkenden Fixierkräfte bei Körpertemperatur des Patienten in der Gelenkauflage'*ein Spannungszustand erzeugt wird, durch den die Belastbarkeit und/oder Haltbarkeit erhöht ist. Hierdurch wird eine in einfacher Weise zu handhabende Fixierung zwischen dem Gelenkplateau und der Gelenkauflage realisiert, die eine wesentlich höhere Belastbarkeit aufgrund des durch die Schrumpfverbindung eingebrachten Spannungszustandes gestattet.

Die Erfindung lässt verschiedene Ausführungsformen zu. Zur weiteren Verdeutlichung ihres Grundprinzips ist eine davon in der Zeichnung dargestellt und wird nachfolgend beschrieben. Diese zeigt in
- Fig.1: eine geschnittene Seitenansicht eines erfindungsgemäßen künstlichen Gelenks;
- Fig.2: eine Detaildarstellung des in Figur 1 gezeigten künstlichen Gelenks;
- Fig.3: eine Draufsicht auf das in Figur 1 gezeigte Gelenk.

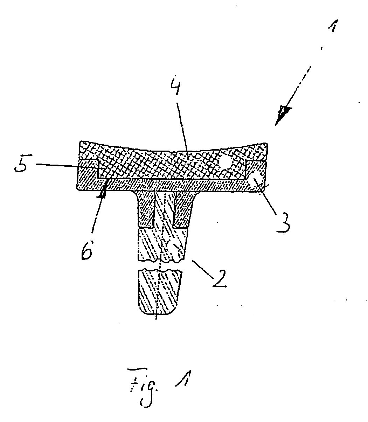

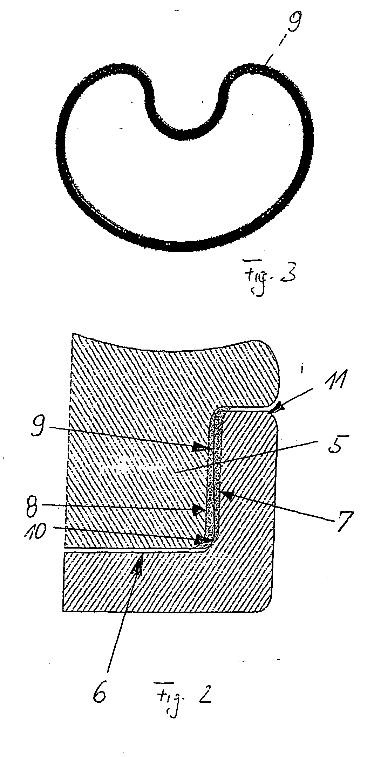

Figur 1 zeigt ein insbesondere als Kniegelenkprothese ausgeführtes künstliches Gelenk 1 in einer geschnittenen Seitenansicht. Zu erkennen ist ein mit einem Zapfen 2 ausgestattetes Gelenkplateau 3, das mit einer Gelenkauflage 4 kraft- und formschlüssig verbunden ist. Hierzu hat die Gelenkauflage 4 einen Vorsprung 5, welcher gegenüber einer Ausnehmung 6 des Gelenkplateaus 3 ein in Figur 2 näher dargestelltes Übermaß 7 aufweist. Um den Vorsprung 5 in die Ausnehmung 6 einsetzen zu können, wird zunächst eine von der Körpertemperatur des Patienten deutlich abweichende Temperatur durch Erwärmung bzw. Abkühlung des Vorsprunges 5 und / oder der Ausnehmung 6 eingestellt und dadurch werden jeweils unterschiedliche thermische Dehnungen erreicht. Nach dem Einsetzen des Vorsprunges 5 in die Ausnehmung 6 erfolgt ein Temperaturausgleich, wodurch sich der Vorsprung 5 in der Ausnehmung 6 verspannt und zugleich flächig anlegt. Die mittels der Temperaturdifferenz realisierte thermische Schrumpfverbindung kann zugleich auch in vorteilhafter Weise dazu genutzt werden, mittels der auf diese Weise eingebrachten Fixierkräfte und dadurch erzeugten Spannungen die Belastbarkeit des Gelenkes 1 zu erhöhen. Hierzu werden die Spannungen in optimaler Weise derart eingestellt, dass diese der lastbedingten Krafteinwirkung entgegenwirken. Beispielsweise kann auf diese Weise ein Spannungszustand erzeugt werden, bei dem die Gelenkauflage 4 lediglich durch Druckkräfte belastet wird, hingegen die Einwirkung von Zugkräften aufgrund der Vorspannung ausgeschlossen ist.

Die genaue Formgebung des Vorsprungs 5 und der Ausnehmung 6 wird anhand der Figur 2 näher dargestellt, die eine Detaildarstellung des in Figur 1 gezeigten künstlichen Gelenks 1 zeigt. Dargestellt ist die gebrauchsbereite Funktionsstellung, in welcher der Vorsprung 5 bereits kraft- und formschlüssig in die Ausnehmung 6 eingesetzt ist. Das ursprüngliche, lediglich gestrichelt dargestellte Übermaß 7 des Vorsprunges 5 wird durch Abkühlung zunächst auf ein ebenfalls gestrichelt dargestelltes Schrumpfmaß 8 reduziert, so dass eine problemlose Montage ermöglicht wird. Bei dem nachfolgenden Temperaturausgleich wird der Vorsprung 5 flächig gegen die Ausnehmung 6 angepresst und dort kraftschlüssig fixiert. Bedingt durch eine geneigte Anordnung einer Kontaktfläche 9 der Ausnehmung 6 ist eine Hinterschneidung 10 gebildet, die zusätzlich zu einer formschlüssigen Fixierung des Vorsprungs 5 in der Ausnehmung 6 führt. Zugleich werden dadurch im Bereich der Hinterschneidung 10 gegenüber einem Randbereich 11 der Ausnehmung 6 voneinander abweichende Spannungsvertäufe erreicht, so dass die resultierende Kraftkomponente zu einer Vorspannung in Richtung des Vorsprunges 5 gegenüber der Ausnehmung 6 führt.

Figur 3 zeigt ergänzend eine Draufsicht auf das in Figur 1 gezeigte Gelenk 1. Zu erkennen ist die umlaufende Kontaktfläche 9 zwischen dem in den Figuren 1 und 2 dargestellten Vorsprung 5 und der Ausnehmung 6. Die Kontaktfläche 9 weist dabei einen stetigen Verlauf auf, durch den eine gleichmäßige Einleitung der Fixierkraft über den gesamten Umfang sichergestellt ist. Hierdurch kann die Belastbarkeit des künstlichen Gelenks 1 weiter gesteigert werden.

## Patentansprüche

1. Ein künstliches Gelenk (1) mit einem Gelenkplateau (3) und einer Gelenkauflage (4), die mittels einer durch eine Ausnehmung (6) und einen darin eingesetzten Vorsprung (5) bestimmte Kontaktfläche (9) miteinander verbunden sind, wobei der Vorsprung (5) gegenüber der Ausnehmung (6) bei Körpertemperatur des Patienten ein Übermaß (7) aufweist und mittels einer Temperaturdifferenz des Vorsprunges (5) und/oder der Ausnehmung (6) gegenüber der Körpertemperatur des Patienten in die Ausnehmung (6) einsetzbar ist, **dadurch gekennzeichnet, dass** die Kontaktfläche (9) bzw. das Übermaß (7) ortsabhängig unterschiedlich derart bemessen ist, dass aufgrund der auf den Vorsprung (5) wirkenden Fixierkräfte bei Körpertemperatur des Patienten in der Gelenkauflage (4) und/oder Gelenkplateaus (3) ein Spannungszustand realisiert ist, durch den die Belastbarkeit und/oder Haltbarkeit des künstlichen Gelenks (1) erhöht ist.

2. Künstliches Gelenk (1) nach Anspruch 1, **dadurch gekennzeichnet, dass** aufgrund der Fixierkräfte ein gezielter Druckzustand in der Gelenkauflage (4) einstellbar ist.

3. Künstliches Gelenk (1) nach den Ansprüchen 1 oder 2, **dadurch gekennzeichnet, dass** die Kontaktfläche (9) derart ausgeführt ist, dass der Spannungszustand oder der Druckzustand hinsichtlich des Betrages und/oder und der Richtung einstellbar ist.

4. Künstliches Gelenk (1) nach zumindest einem der vorhergehenden Ansprüche, **dadurch gekennzeichnet, dass** der Betrag des aufgrund der Fixierkräfte eingebrachten Spannungszustandes oder Druckzustandes größer als der Betrag einer äußeren Krafteinwirkung ist.

5. Künstliches Gelenk (1) nach zumindest einem der vorhergehenden Ansprüche, **dadurch gekennzeichnet, dass** der Druck- bzw. Spannungszustand in Abhängigkeit der Hauptbelastungsebene des künstlichen Gelenkes (1) einstellbar ist.

6. Künstliches Gelenk (1) nach zumindest einem der vorhergehenden Ansprüche, **dadurch gekennzeichnet, dass** der Spannungszustand durch eine einer äußeren Krafteinwirkung entgegenwirkende Vorspannung gegeben ist.

7. Künstliches Gelenk (1) nach zumindest einem der vorhergehenden Ansprüche, **dadurch gekennzeichnet, dass** im Bereich der Kontaktfläche ein zur Einstellung des Spannungszustandes vorgesehenes Zwischenelement vorgesehen ist.

8. Künstliches Gelenk (1) nach Anspruch 6, **dadurch gekennzeichnet, dass** das Zwischenelement in unterschiedlichen Positionen festlegbar ist.

9. Künstliches Gelenk (1) nach Anspruch 6 oder 7, **dadurch gekennzeichnet, dass** das Zwischenelement als eine Schale ausgeführt ist.

10. Künstliches Gelenk (1) nach zumindest einem der vorhergehenden Ansprüche, **dadurch gekennzeichnet, dass** die Ausnehmung (6) eine den Vorsprung (5) formschlüssig fixierende Hinterschneidung (10) aufweist, die durch eine Kontur oder eine Topographie der Gelenkauflage (4), insbesondere der Materialstärke, bestimmt ist.

11. Künstliches Gelenk (1) nach zumindest einem der vorhergehenden Ansprüche, **dadurch gekennzeichnet, dass** eine das Gelenkplateau (3) und die Gelenkauflage (4) jeweils einen unterschiedlichen Wärmeausdehnungskoeffizienten aufweisen.

12. Künstliches Gelenk (1) nach zumindest einem der vorhergehenden Ansprüche, **dadurch gekennzeichnet, dass** eine das Gelenkplateau (3) und die Gelenkauflage (4) konturbündig miteinander verbunden sind.

13. Künstliches Gelenk (1) nach zumindest einem der vorhergehenden Ansprüche, **dadurch gekennzeichnet, dass** eine Kontaktfläche (9) zwischen dem Vorsprung (5) und der Ausnehmung (6) mit einer die Kraftübertragung verbessernden Oberflächenbeschaffenheit, insbesondere Rauhigkeit oder Strukturierung, ausgestattet ist.

14. Künstliches Gelenk (1) nach zumindest einem der vorhergehenden Ansprüche, **dadurch gekennzeichnet, dass** der Gelenkauflage (4) ein Vorsprung (5) aus Polyethylen und dem Gelenkplateau aus einem Metall (3) eine Ausnehmung (6) zugeordnet ist.

15. Künstliches Gelenk (1) nach zumindest einem der vorhergehenden Ansprüche, **dadurch gekennzeichnet, dass** der Vorsprung (5) und die Ausnehmung (6) an einer umlaufenden Kontaktfläche (9) gegeneinander liegen, die einen stetigen Verlauf hat.

16. Verfahren zum Verbinden eines Gelenkplateau mit einer Gelenkauflage eines künstlichen Gelenks nach Anspruch 1, bei dem das Gelenkplateau mit der Gelenkauflage mittels einer durch eine Ausnehmung und einen darin eingesetzten Vorsprung gebildeten Kontaktfläche verbunden wird, wobei zunächst mittels einer Temperaturdifferenz gegenüber der Körpertemperatur des Patienten eine unterschiedliche Wärmedehnung und / oder Schrumpfung zwischen dem Vorsprung und der Ausnehmung erreicht und anschließend der Vorsprung in die Ausnehmung eingesetzt wird, **dadurch gekennzeichnet, dass** die Kontaktfläche bzw. das Übermaß ortsabhängig unterschiedlich derart bemessen werden, dass aufgrund der auf den Vorsprung wirkenden Fixierkräfte bei Körpertemperatur des Patienten in der Gelenkauflage ein Spannungszustand erzeugt wird, durch den die Belastbarkeit und/oder Haltbarkeit erhöht ist.

## Claims

1. An artificial joint (1) with a joint plateau (3) and a joint overlay (4), which are connected to one another by means of a contact face (9) determined by a recess (6) and a projection (5) inserted therein, wherein the projection (5) has an over-dimension (7) compared to the recess (6) at the body temperature of the patient and can be inserted into the recess (6) by virtue of a temperature difference of the projection (5) and/or the recess (6) relative to the body temperature of the patient, **characterised in that** the contact face (9) or the over-dimension (7), depending on the site, has different dimensions such that because of the securing forces acting on the projection (5), at the body temperature of the patient, a stress state is produced in the joint overlay (4) and/or joint plateau (3) by means of which the load-bearing capacity and/or durability of the artificial joint (1) is increased.

2. Artificial joint (1) according to claim 1, **characterised in that** a specific pressure state can be adjusted in the joint overlay (4) because of the securing forces.

3. Artificial joint (1) according to claims 1 or 2, **characterised in that** the contact face (9) is designed such that the stress state or the pressure state can be adjusted with regard to the amount and/or the direction.

4. Artificial joint (1) according to at least any one of the preceding claims, **characterised in that** the amount of the stress state or pressure state introduced because of the securing forces is greater than the amount of an external application of force.

5. Artificial joint (1) according to at least any one of the preceding claims, **characterised in that** the pressure or stress state can be adjusted as a function of the main loading plane of the artificial joint (1).

6. Artificial joint (1) according to at least any one of the preceding claims, **characterised in that** the stress state is provided by a prestressing counteracting an external application of force.

7. Artificial joint (1) according to at least any one of the preceding claims, **characterised in that** an intermediate element provided to adjust the stress state is provided in the region of the contact face.

8. Artificial joint (1) according to claim 6, **characterised in that** the intermediate element can be fixed in different positions.

9. Artificial joint (1) according to claim 6 or 7, **characterised in that** the intermediate element is designed as a bowl.

10. Artificial joint (1) according to at least any one of the preceding claims, **characterised in that** the recess (6) has an undercut (10) securing the projection (5) in a positive manner, which undercut is determined by a contour or a topography of the joint overlay (4), in particular the material thickness.

11. Artificial joint (1) according to at least any one of the preceding claims, **characterised in that** the joint plateau (3) and the joint overlay (4) in each case have different coefficients of thermal expansion.

12. Artificial joint (1) according to at least any one of the preceding claims, **characterised in that** the joint plateau (3) and the joint overlay (4) are connected to one another with the contours flush.

13. Artificial joint (1) according to at least any one of the preceding claims, **characterised in that** a contact face (9) between the projection (5) and the recess (6) is equipped with a surface characteristic improving the force transmission, in particular roughness or structuring.

14. Artificial joint (1) according to at least any one of the preceding claims, **characterised in that** a projection (5) made of polyethylene is associated with the joint overlay (4) and a recess (6) is associated with the joint plateau made of a metal (3).

15. Artificial joint (1) according to at least any one of the preceding claims, **characterised in that** the projection (5) and the recess (6) rest against one another on a peripheral contact face (9), which has a constant course.

16. Method for connecting a joint plateau to a joint overlay of an artificial joint according to claim 1, in which the joint plateau is connected to the joint overlay by means of a contact face formed by a recess and a projection inserted therein, wherein a different thermal expansion and/or shrinkage is firstly achieved between the projection and the recess by virtue of a temperature difference relative to the body temperature of the patient and the projection is then inserted into the recess, **characterised in that** the contact face or the over-dimension, depending on the site, have different dimensions in such a way that because of the securing forces acting on the projection at the body temperature of the patient, a stress state is produced in the joint overlay, by means of which the load-bearing capacity and/or durability is increased.

## Revendications

1. Articulation artificielle (1) comportant un plateau d'articulation (3) et un revêtement d'articulation (4), qui sont reliés l'un à l'autre grâce à une surface de contact (9) déterminée par un évidement (6) et une saillie (5) insérée à l'intérieur, moyennant quoi la saillie (5) présente un surdimensionnement (7) par rapport à l'évidement (6), à la température corporelle du patient, et peut être insérée dans l'évidement (6) grâce à la différence de température de la saillie (5) et/ou de l'évidement (6) par rapport à la température corporelle du patient, **caractérisée en ce que** la surface de contact (9) ou le surdimensionnement (7) est mesuré(e) de manière différente en fonction de l'emplacement de telle sorte que, en raison des forces de fixation exercées sur la saillie (5), à la température corporelle du patient, un état de tension est obtenu dans le revêtement d'articulation (4) et/ou le plateau d'articulation (3), grâce auquel l'aptitude à la sollicitation et/ou la stabilité de l'articulation artificielle (1) sont accrues.

2. Articulation artificielle (1) selon la revendication 1, **caractérisée en ce que**, en raison des forces de fixation, un état de pression ciblé peut être réglé dans le revêtement d'articulation (4).

3. Articulation artificielle (1) selon la revendication 1 ou 2, **caractérisée en ce que** la surface de contact (9) est réalisée de telle sorte que l'état de tension ou l'état de pression peut être réglé quant au degré et/ou à la direction.

4. Articulation artificielle (1) selon au moins l'une quelconque des revendications précédentes, **caractérisée en ce que** le degré de l'état de tension ou de l'état de pression créé en raison des forces de fixation est supérieur au degré d'une force externe exercée.

5. Articulation artificielle (1) selon au moins l'une quelconque des revendications précédentes, **caractérisée en ce que** l'état de pression ou de tension peut être réglé en fonction du niveau de sollicitation principale de l'articulation artificielle (1).

6. Articulation artificielle (1) selon au moins l'une quelconque des revendications précédentes, **caractérisée en ce que** l'état de tension est défini par une précontrainte opposée à l'exercice d'une force externe.

7. Articulation artificielle (1) selon au moins l'une quelconque des revendications précédentes, **caractérisée en ce que**, dans la zone de la surface de contact, il est prévu un élément intermédiaire permettant de régler l'état de tension.

8. Articulation artificielle (1) selon la revendication 6, **caractérisée en ce que** l'élément intermédiaire peut être fixé dans différentes positions.

9. Articulation artificielle (1) selon la revendication 6 ou 7, **caractérisée en ce que** l'élément intermédiaire est configuré sous la forme d'une coque.

10. Articulation artificielle selon au moins l'une quelconque des revendications précédentes, **caractérisée en ce que** l'évidement (6) présente une contre-dépouille (10) fixant la saillie (5) par conjugaison de forme, laquelle contre-dépouille (10) est déterminée par un contour ou une topographie du revêtement de l'articulation (4), en particulier la résistance du matériau.

11. Articulation artificielle (1) selon au moins l'une quelconque des revendications précédentes, **caractérisée en ce que** le plateau de l'articulation (3) et le revêtement de l'articulation (4) présentent respectivement des coefficients de dilatation thermique différents.

12. Articulation artificielle (1) selon au moins l'une quelconque des revendications précédentes, **caractérisée en ce que** le plateau d'articulation (3) et le revêtement d'articulation (4) sont reliés l'un à l'autre en alignant les contours.

13. Articulation artificielle (1) selon au moins l'une quelconque des revendications précédentes, **caractérisée en ce qu'**une surface de contact (9) est dotée, entre la saillie (5) et l'évidement (6), d'une texture superficielle qui améliore la transmission des forces, en particulier rugosité ou structuration.

14. Articulation artificielle (1) selon au moins l'une quelconque des revendications précédentes, **caractérisée en ce que**, au revêtement d'articulation (4), est associée une saillie (5) constituée de polyéthylène et, au plateau d'articulation (4) réalisé en métal (3), est associé un évidement (6).

15. Articulation artificielle (1) selon au moins l'une quelconque des revendications précédentes, **caractérisée en ce que** la saillie (5) et l'évidement (6) se trouvent face à face sur une surface de contact (9) circulaire, qui présente une évolution régulière.

16. Procédé permettant de relier un plateau d'articulation à un revêtement d'articulation d'une articulation artificielle selon la revendication 1, dans lequel le plateau d'articulation est relié au revêtement d'articulation à l'aide d'une surface de contact formée par un évidement et une saillie insérée à l'intérieur, moyennant quoi, tout d'abord à l'aide d'une différence de température par rapport à la différence corporelle du patient, on atteint une dilatation thermique et/ou une contraction différente entre la saillie et l'évidement, puis la saillie est insérée dans l'évidement, **caractérisé en ce que** la surface de contact ou le surdimensionnement est mesuré(e) différemment en fonction de l'emplacement, de telle sorte que, en raison des forces de fixation s'exerçant sur la saillie, à la température corporelle du patient, un état de tension est produit dans le revêtement d'articulation, grâce auquel l'aptitude à la sollicitation et/ou la stabilité sont accrues.
